(19) Europäisches Patentamt
European Patent Office
Office européen des brevets

(11) Veröffentlichungsnummer: **0 032 672**
**B1**

# (12) EUROPÄISCHE PATENTSCHRIFT

(45) Veröffentlichungstag der Patentschrift: **21.09.83**

(51) Int. Cl.³: **C 07 C 101/54**, C 07 C 99/00

(21) Anmeldenummer: **81100065.2**

(22) Anmeldetag: **08.01.81**

(54) **Verfahren zur Herstellung von Anthranilsäurealkylestern.**

(30) Priorität: **17.01.80 DE 3001579**

(43) Veröffentlichungstag der Anmeldung:
**29.07.81 Patentblatt 81/30**

(45) Bekanntmachung des Hinweises auf die Patenterteilung:
**21.09.83 Patentblatt 83/38**

(84) Benannte Vertragsstaaten:
**CH DE FR GB IT LI NL**

(56) Entgegenhaltungen:
EP-A-2 624
DE-A-375 616
DE-A-2 834 168

(73) Patentinhaber: **BASF Aktiengesellschaft,**
**Carl-Bosch-Strasse 38, D-6700 Ludwigshafen (DE)**

(72) Erfinder: **Reissenweber, Gernot, Dr.,**
**Pfarrer-Friedrich-Strasse 41, D-6700 Ludwigshafen (DE)**
Erfinder: **Mangold, Dietrich, Dr.,**
**Hermann-Walker-Strasse 49, D-6903 Neckargemuend (DE)**

EP 0 032 672 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Anthranilsäurealkylestern

Die Erfindung betrifft ein neues Verfahren zur Herstellung von Anthranilsäurealkylestern durch Umsetzung von Isatinen mit Alkanolen und Wasserstoffperoxid in Gegenwart von Alkalialkanolaten.

Es ist bekannt, dass man durch Veresterung von Anthranilsäuren die entsprechenden Anthranilsäureester erhält [Rodd, Chemistry of Carbon Compounds, Band IIIa, Seite 578 (Elsevier, N.Y. 1954)]. Ebenso erhält man Anthranilsäureester durch Umsetzung von Isatosäureanhydriden mit Alkoholen in Gegenwart von Natriumhydroxid [J. Org. Chem., Band 24, Seiten 1214-1219 (1959)] oder mit alkoholischen Alkoholatlösungen. Phthalimid ergibt durch Umsetzung mit einer alkoholischen Lösung von Hypochlorit ebenfalls diese Ester (Rodd, loc. cit.). Ein weiterer Weg zu Anthranilsäureestern führt über die Nitrierung von Benzoesäurederivaten mit anschliessender Reduktion der Nitrogruppe und nachfolgender Veresterung (Houben-Weyl, Methoden der Organischen Chemie, Band 11/1, Seite 367).

Diese Verfahren sind nachteilig: Spezifisch substituierte Anthranilsäuren bzw. Isatosäureanhydride sind nur schwer erhältlich und werden in der Regel aus entsprechend substituierten Isatinen hergestellt. Durch Oxidation von Isatinen mit Wasserstoffperoxid in verdünnter, wässriger Natronlauge erhält man bei 10 bis 15°C Anthranilsäuren (Houben-Weyl, loc. cit., Band 7/4, Seiten 30 und 31) und durch Oxidation mit Chromatrioxid in Essigsäure die entsprechenden Isatosäureanhydride [J. Org. Chem., Band 17, Seite 173 (1952)], so dass insgesamt ein Reaktionsschritt mehr benötigt wird. Bei der Nitrierung von Benzoesäurederivaten erhält man in der Regel ein schwer zu trennendes Produktgemisch. So liefert z.B. die Nitrierung von 3-Methylbenzoesäure ein Gemisch aus 2-Nitro-, 4-Nitro- und 6-Nitro-3-methylbenzoesäure [Chem. Ber., Band 42, Seiten 430 und 431 (1909)].

Die EU-A-0 002 624 zeigt ein Verfahren der Oxidation von Isatinen in saurer Lösung in Abwesenheit von Alkanolen mit m-Chlorperbenzoesäure zu Isatosäureanhydriden (siehe auch Seite 2, Formelschema; Beispiel 1, Zeilen 1 bis 4). Erst in einem zweiten Reaktionsschritt werden die Isatosäureanhydride in Methanol/Natriummethylat zu Anthranilsäureester umgesetzt.

Es wurde nun gefunden, dass man Anthranilsäurealkylester der Formel

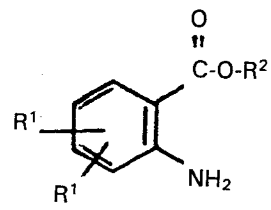

(I)

worin die einzelnen Reste $R^1$ gleich oder verschieden sein können und jeweils ein Wasserstoffatom, ein Halogenatom, einen Alkylrest oder halogenierten Alkylrest mit 1 bis 18 Kohlenstoffatomen und gegebenenfalls 1 bis 3 Chlor-, Brom- und/oder Fluoratomen, den Rest $-OR^3$, worin $R^3$ einen Alkylrest mit 1 bis 18 Kohlenstoffatomen bedeutet, oder eine Nitrogruppe bezeichnen, $R^2$ für einen Alkylrest mit 1 bis 18 Kohlenstoffatomen steht, vorteilhaft erhält, wenn man Isatine der Formel

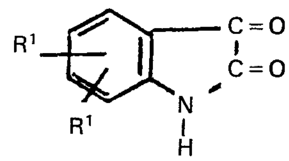

II

worin $R^1$ die vorgenannte Bedeutung besitzt, mit Alkanolen der Formel

$$R^2OH \qquad III$$

worin $R^2$ die vorgenannte Bedeutung besitzt, und wässerigem Wasserstoffperoxid in Gegenwart von Alkalialkanolaten der Formel

$$ZOR^2 \qquad IV$$

worin Z ein Alkaliatom bezeichnet und $R^2$ die vorgenannte Bedeutung besitzt, umsetzt.

Die Umsetzung kann für den Fall der Verwendung von Isatin und Methanol durch die folgenden Formeln wiedergegeben werden:

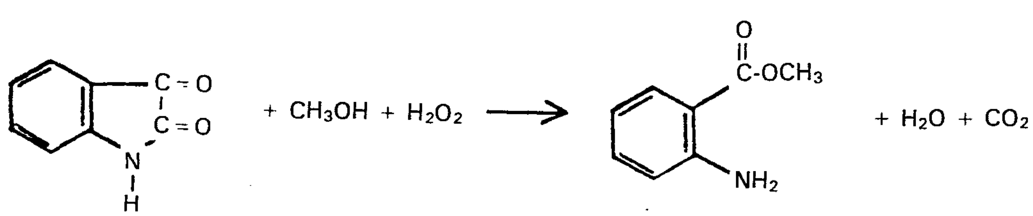


Im Vergleich zu den bekannten Verfahren liefert das Verfahren nach der Erfindung auf einfacherem und wirtschaftlicherem Wege Anthranilsäurealkylester in guter Ausbeute und Reinheit und, wenn man die Herstellung der Ausgangsstoffe in Betracht zieht, besserer Raum-Zeit-Ausbeute. Alle diese vorteilhaften Ergebnisse sind im Hinblick auf den Stand der Technik überraschend.

Die Ausgangsstoffe können miteinander in stöchiometrischer Menge oder jeder im Überschuss zum anderen, vorzugsweise in einer Menge von 40 bis 100, insbesondere 60 bis 80 Mol Alkanol III und/oder

1 bis 3, insbesondere 1 bis 1,5 Mol Wasserstoffperoxid je Mol Isatin II umgesetzt werden. Bevorzugte Ausgangsstoffe II, III und dementsprechend bevorzugte Endstoffe I sind solche, in deren Formeln die einzelnen Reste $R^1$ gleich oder verschieden sein können und jeweils ein Wasserstoffatom, ein Bromatom, ein Chloratom, einen Alkylrest mit 1 bis 6 Kohlenstoffatomen, einen halogenierten Alkylrest mit 1 bis 6 Kohlenstoffatomen und 1, 2 oder 3 Chloratomen, Bromatomen und/oder Fluoratomen, den Rest $-OR^3$, worin $R^3$ einen Alkylrest mit 1 bis 6 Kohlenstoffatomen bedeutet, oder eine Nitrogruppe bezeichnen, $R^2$ für einen Alkylrest mit 1 bis 6 Kohlenstoffatomen steht.

Beispielsweise sind folgende Isatine als Ausgangsstoffe II geeignet: Isatin; 4-, 5-, 6- oder 7-Stellung einfach oder in 4,5-, 5,6-, 6,7-, 4,6-, 5,7-, 4,7-Stellung gleich oder unterschiedlich zweifach durch ein Bromatom, Chloratom, Methyl-, Äthyl-, Propyl-, Isopropyl-, Butyl-, Isobutyl-, sek.-Butyl-, tert.-Butyl-, Methoxy-, Äthoxy-, Propoxy-, Isopropoxy-, Butoxy-, Isobutoxy-, sek.-Butoxy-, tert.-Butoxy-, Trifluormethyl-, Nitro-gruppe substituierte Isatine.

Es kommen z.B. folgende Alkanole als Ausgangsstoffe III in Frage: Methyl-, Äthyl-, Propyl-, Butyl-, Isopropyl-, sek.-Butyl-, tert.-Butyl-, Isobutylalkohol; bevorzugt sind Methanol und Äthanol.

Das Wasserstoffperoxid wird zweckmässig in Form seiner 5- bis 60-, vorzugsweise 20- bis 55-gewichtsprozentigen, wässrigen Lösung verwendet. Gegebenenfalls kommen auch Stoffe in Betracht, die unter den Umsetzungsbedingungen Wasserstoffperoxid bilden, z.B. anorganische oder organische Peroxoverbindungen wie Natriumperoxid, Kaliumperoxid, Magnesiumperoxid, Calciumperoxid, Zinkperoxid, Bariumperoxid, Bariumsuperoxid; Hydroperoxide wie $NaOOH \cdot 0,5\ H_2O_2$, $NH_4OOH$; entsprechende Hydrate wie $CaO_2 \cdot 8H_2O$, Peroxohydrate wie $BaO_2 \cdot H_2O_2$ und $BaO_2 \cdot 2\ H_2O_2$; Natriumperoxodisulfat, Kaliumperoxodisulfat, Ammoniumperoxodisulfat. Ebenfalls können Wasserstoffperoxidanlagerungsverbindungen verwendet werden wie Natriumboratperoxohydrat. Gegebenenfalls können Hilfsstoffe wie Magnesiumsulfat oder Magnesiumchlorid zugesetzt werden.

Als Alkalialkanolate IV werden bevorzugt solche verwendet, in deren Formeln $R^2$ die vorgenannte bevorzugte Bedeutung besitzt; geeignet sind z.B. die Alkalialkanolate der als beispielsweise geeignet vorgenannten Alkanole III. In der Regel wird man gleichzeitig ein bestimmtes Alkanol III und sein entsprechendes Alkalialkanolat IV verwenden. Bevorzugte Alkanolate sind das Kaliumalkanolat und Natriumalkanolat. Die Alkanolate IV können in stöchiometrischer Menge oder im Überschuss, vorzugsweise in einer Menge von 1 bis 5, insbesondere 2 bis 3 Mol Alkanolat IV je Mol Ausgangsstoff II verwendet werden. Anstelle der Alkanolate IV können auch Stoffe, die unter den Reaktionsbedingungen Alkanolate bilden, z.B. Alkanol und Alkalihydrid, verwendet werden.

Die Umsetzung wird zweckmässig bei einer Temperatur von $-50$ bis $+80\,°C$, vorzugsweise $-10$ bis $+50\,°C$, drucklos oder unter Druck, kontinuierlich oder diskontinuierlich durchgeführt. Zweckmässig wird man im Falle der flüssigen Alkanole III, insbesondere von Alkanolen mit 1 bis 4 Kohlenstoffatomen, das Reaktionsgemisch als Lösungsmedium verwenden und kein weiteres Lösungsmittel zusetzen. Gegebenenfalls verwendet man unter den Reaktionsbedingungen inerte Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdecan, 1,4-Dibrombutan; Nitrokohlenwasserstoffe wie Nitromethan, Nitroäthan, Nitrobenzol, o-, m-, p-Chlornitrobenzol, o-Nitrotoluol; Sulfoxide wie Dimethylsulfoxid, Diäthylsulfoxid, Dimethylsulfon, Diäthylsulfon, Methyläthylsulfon, Tetramethylensulfon; Dimethylformamid; und entsprechende Gemische. Zweckmässig verwendet man das Lösungsmittel in einer Menge von 400 bis 10000 Gewichtsprozent, vorzugsweise von 600 bis 1000 Gewichtsprozent, bezogen auf Ausgangsstoff II.

Die Reaktion kann wie folgt durchgeführt werden: Wasserstoffperoxid, die Ausgangsstoffe II, III und Stoff IV, gegebenenfalls im Gemisch mit Lösungsmitteln, werden bei der Reaktionstemperatur während 0,5 bis 2 Stunden gehalten. Dann wird der Endstoff aus dem Gemisch in üblicher Weise, z.B. durch fraktionierte Destillation, abgetrennt. Der Endstoff kann auch in folgender Weise isoliert werden: Nach Destillation des Lösungsmittels wird der Rückstand in Wasser aufgenommen und neutral gestellt, die wässrige Lösung wird mit Methylenchlorid extrahiert und der Extrakt destilliert.

Die nach dem Verfahren der Erfindung erhältlichen Anthranilsäurealkylester der Formel I sind wertvolle Ausgangsstoffe für die Herstellung von Schädlingsbekämpfungsmitteln, Farbstoffen und Pharmazeutika. So erhält man beispielsweise durch Umsetzung von Anthranilsäureestern mit Aminosulfonylchloriden und nachfolgender Cyclisierung die als Herbizide bekannten Benzothiadiazone (DOS 2 443 901). Ebenfalls kann man z.B. aus ihnen durch Umsetzung der Anthranilsäureester I mit Sulfamidsäurechloriden die in der deutschen Offenlegungsschrift 2 104 682 beschriebenen o-Sulfamidobenzoesäuren herstellen. Durch Cyclisierung dieser Stoffe, z.B. nach dem in der deutschen Offenlegungsschrift 2 105 687 beschriebenen Verfahren, gelangt man zu den 2,1,3-Benzothiadiazin-4-on-2,2-dioxiden, deren Verwendung für Pflanzenschutzmittel und Pharmazeutika in derselben Patentschrift beschrieben ist. Die sehr guten herbiziden Eigenschaften dieser Verbindungsklasse sind in der US-Patentschrift 3 621 017 sowie in der deutschen Patentschrift 1 937 551 und der deutschen Offenlegungsschrift 2 131 401 beschrieben.

Weiterhin können die Endstoffe I als Zusätze in Parfümen, Sonnenbrandölen, Brandsalben und als Alterungsschutz für Synthesekautschuk Verwendung finden. Bezüglich weiterer Verwendung wird auf die vorgenannten Veröffentlichungen und Ullmanns Encyklopädie der technischen Chemie, Band 8, Seite 375, verwiesen.

Die in den folgenden Beispielen aufgeführten Teile bedeuten Gewichtsteile. Sie verhalten sich zu Volumenteilen wie Kilogramm zu Liter.

*Beispiel 1*

Eine Suspension von 14,7 Teilen Isatin in 200 Teilen Methanol wird bei 0°C mit 20 Teilen 30gewichtsprozentiger, methanolischer Natriummethylatlösung versetzt. Anschliessend gibt man unter Kühlung bei −3°C 8 Teile 50gewichtsprozentige, wässrige Wasserstoffperoxidlösung zu, wobei sich die zunächst dunkelviolette Lösung entfärbt. Man lässt das Gemisch 30 Minuten bei Raumtemperatur nachrühren, dampft das Lösungsmittel ab, versetzt den Rückstand mit Wasser und schüttelt das Gemisch mit Methylenchlorid aus. Nach Verdampfen des Methylenchlorids erhält man 12,4 Teile (82% der Theorie) Anthranilsäuremethylester vom Fp 21 bis 23°C.

*Beispiel 2*

Eine Lösung aus 32,2 Teilen 7-Methylisatin und 12 Teilen Natriummethylat in 400 Teilen Methanol wird bei 0°C langsam mit 15 Teilen 50gewichtsprozentiger Wasserstoffperoxidlösung versetzt. Nach Zugabe des Wasserstoffperoxids wird das Gemisch 2 Stunden bei Raumtemperatur nachgerührt und wie in Beispiel 1 beschrieben aufgearbeitet. Man erhält 25,5 Teile (78% der Theorie) 3-Methylanthranilsäuremethylester vom Fp 27 bis 29°C.

*Beispiel 3*

21,6 Teile 5,7-Dichlorisatin in 300 Teilen Methanol werden bei −10°C mit 20 Teilen 30gewichtsprozentiger, methanolischer Natriummethylatlösung versetzt, wobei eine dunkelviolette voluminöse Suspension entsteht. Anschliessend gibt man bei −3°C 7 Teile 50gewichtsprozentige Wasserstoffperoxidlösung zu und rührt das Gemisch eine Stunde bei Raumtemperatur nach. Die Lösung wird eingeengt, der kristalline Rückstand mit Wasser versetzt und abgesaugt. Man erhält 17,6 Teile (80% der Theorie) 3,5-Dichloranthranilsäuremethylester vom Fp 62 bis 63°C.

*Beispiel 4*

Analog Beispiel 3 erhält man aus 22,6 Teilen 5-Bromisatin 17,4 Teile (76% der Theorie) 5-Bromanthranilsäuremethylester vom Fp 73 bis 74°C.

*Beispiel 5*

19,2 Teile 5-Nitroisatin in 300 Teilen Methanol werden bei 0°C nacheinander mit 20 Teilen 30gewichtsprozentiger, methanolischer Natriummethylatlösung und 8 Teilen 50gewichtsprozentiger Wasserstoffperoxidlösung versetzt. Nach Aufarbeitung, wie in Beispiel 3 beschrieben, erhält man 16 Teile (82% der Theorie) 5-Nitroanthranilsäuremethylester vom Fp 166 bis 168°C.

*Beispiel 6*

21,5 Teile 7-Trifluormethylisatin in 300 Teilen Methanol werden bei 0°C mit 20 Teilen 30gewichtsprozentiger, methanolischer Natriummethylatlösung versetzt, wobei eine violette Lösung entsteht. Anschliessend gibt man bei 0°C 8 Teile 50gewichtsprozentige Wasserstoffperoxidlösung zu und rührt das Gemisch eine Stunde bei Raumtemperatur nach. Die rötliche Lösung wird eingeengt, mit Wasser versetzt, anschliessend mit Salzsäure auf pH 8 eingestellt und mit Methylenchlorid ausgeschüttelt. Nach Einengen der Methylenchloridphase erhält man 16,2 Teile (74% der Theorie) 3-Trifluormethylanthranilsäuremethylester.

$H^1$-NMR ($CDCl_3$): $\delta$ = 3,8 ppm (s); 6,4 (s, $N\text{-}H_2$); 6,6 (t); 7,5 (d); 7,95 (d).

*Beispiel 7*

14,7 Teile Isatin in 250 Teilen n-Butanol und 100 Teilen Dimethylformamid werden bei 25°C mit 8,6 Teilen 50gewichtsprozentigem Natriumhydrid versetzt. Nach einer Stunde gibt man in die violette Lösung langsam 8 Teile 50gewichtsprozentige Wasserstoffperoxidlösung und lässt die Temperatur bis auf 50°C steigen. Anschliessend lässt man eine Stunde nachrühren und arbeitet, wie in Beispiel 1 beschrieben, auf. Man erhält 10,8 Teile (56% der Theorie) Anthranilsäure-n-butylester.

$H^1$-NMR ($CDCl_3$): $\delta$ = 0,8-1,8 ppm (m, 7H); 4,1 (t, 2H); 6,2-7,6 (m, 5H); 6,4 (s, $NH_2$).

**Patentanspruch**

Verfahren zur Herstellung von Anthranilsäurealkylestern der Formel

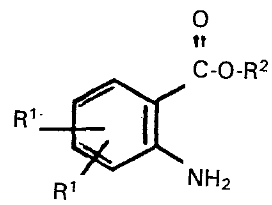

(I)

worin die einzelnen Reste $R^1$ gleich oder verschieden sein können und jeweils ein Wasserstoffatom, ein Halogenatom, einen Alkylrest oder halogenierten Alkylrest mit 1 bis 18 Kohlenstoffatomen und gegebenenfalls 1 bis 3 Chlor-, Brom- und/oder Fluoratomen, den Rest $-OR^3$, worin $R^3$ einen Alkylrest mit 1 bis 18 Kohlenstoffatomen bedeutet, oder eine Nitrogruppe bezeichnen, $R^2$ für einen Alkylrest mit 1 bis 18 Kohlenstoffatomen steht, dadurch gekennzeichnet, dass man Isatine der Formel

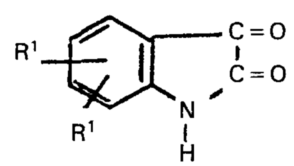

II

worin $R^1$ die vorgenannte Bedeutung besitzt, mit Alkanolen der Formel

$R^2OH$ III

worin $R^2$ die vorgenannte Bedeutung besitzt, und wässerigem Wasserstoffperoxid in Gegenwart von Alkalialkanolaten der Formel

$ZOR^2$ IV

worin Z ein Alkaliatom bezeichnet und $R^2$ die vorgenannte Bedeutung besitzt, umsetzt.

**Claim**

A process for the preparation of alkyl anthranilates of the formula

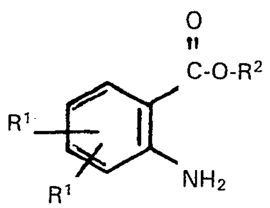

(I)

where the $R^1$'s may be identical or different and each is hydrogen, halogen, alkyl or halogenated alkyl having 1 to 18 carbon atoms and optionally containing 1 to 3 chlorine, bromine and/or fluorine atoms, $-OR^3$ (where $R^3$ is alkyl of 1 to 18 carbon atoms) or nitro, and $R^2$ is alkyl of 1 to 18 carbon atoms, wherein an isatin of the formula

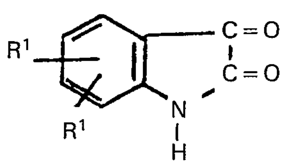

II

where $R^1$ has the above meanings, is reacted with an alkanol of the formula

$R^2OH$ III

where $R^2$ has the above meaning, and aqueous hydrogen peroxide in the presence of an alkali metal alkanolate of the formula

$ZOR^2$ IV

where Z is an alkali metal atom and $R^2$ has the above meaning.

**Revendication**

Procédé de préparation d'esters alkyliques d'acides anthraniliques de formule

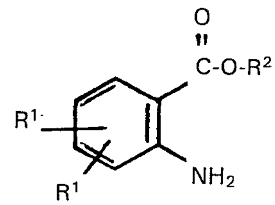

(I)

dans laquelle les divers symboles $R^1$, pouvant avoir des significations identiques ou différentes, représentent chacun un atome d'hydrogène, un atome d'halogène, un groupe alkyle ou un groupe alkyle halogéné en C1-C18 portant éventuellement 1 à 3 atomes de chlore, de brome et/ou de fluor, le reste $-OR^3$ dans lequel $R^3$ représente un groupe alkyle en C1-C18 ou bien un groupe nitro, $R^2$ représente un groupe alkyle en C1-C18, caractérisé en ce que l'on fait réagir des isatines de formule

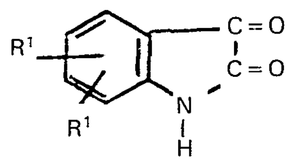

II

dans laquelle $R^1$ a les significations indiquées ci-dessus, avec des alcanols de formule

$R^2OH$ III

dans laquelle $R^2$ a la signification indiquée ci-dessus, et du peroxyde d'hydrogène aqueux en présence d'alcanolates alcalin de formule

$ZOR^2$ IV

dans laquelle Z représente un atome de métal alcalin et $R^2$ a la signification indiquée ci-dessus.